# EUROPÄISCHE PATENTSCHRIFT

(11) **EP 1 023 106 B1**
(45) Veröffentlichungstag und Bekanntmachung des Hinweises auf die Patenterteilung: **26.12.2007**
(21) Anmeldenummer: 98958814.0
(22) Anmeldetag: 16.10.1998
(51) Int. Cl.: A61N 2/02

(54) **VORRICHTUNG ZUR MAGNETFELDTHERAPIE**
DEVICE FOR MAGNETIC FIELD THERAPY
DISPOSITIF DE MAGNETOTHERAPIE

(30) Priorität: 17.10.1997 DE 29718337 U
(43) Veröffentlichungstag der Anmeldung: 02.08.2000
(60) Teilanmeldung: 07013360.8
(73) Patentinhaber: Muntermann, Axel, 35583 Wetzlar (DE)
(72) Erfinder: Muntermann, Axel, 35583 Wetzlar (DE)
(74) Vertreter: Herden, Andreas F.
(86) Internationale Anmeldenummer: PCT/DE1998/003033
(87) Internationale Veröffentlichungsnummer: WO 1999/020345

(56) Entgegenhaltungen:
- EP-A- 0 407 006
- WO-A-96/39493
- DE-A- 4 221 739
- US-A- 5 312 534
- US-A- 5 441 495

## Beschreibung

Die Erfindung betrifft eine Vorrichtung zur Magnetfeldtherapie von menschlichem Gewebe, deren Organen oder Zellgruppen, welche zumindest eine Einrichtung zur Erzeugung eines magnetischen Feldes mit einer statischen und einer Wechselfeld-Komponente am Ort des zu behandelnden Gewebes umfaßt.

Die kurative, aber auch die prophylaktische Magnetfeldbehandlung ist auf dem Gebiet wohlbekannt. Während anfänglich das zu behandelnde Gewebe dem Magnetfeld eines Dauermagneten ausgesetzt wurde, werden nun für die Erzeugung der Felder häufig stromdurchflossene Spulen verwendet. In EP 0594655 B1 ist beispielsweise eine Vorrichtung mit einem niederfrequent gepulste elektrische Ströme erzeugenden Generator und einer darin angeschlossenen Sendespule offenbart, deren elektromagnetischen Felder zur Beaufschlagung einer zu behandelnden Körperregion dienen. Um eine Vorrichtung zum Transport von Ionen und insbesondere von Protonen bereit zu stellen, welche eine gezielte Beeinflussung der Ionenbeweglichkeit in beliebigen Körperbereichen bei Menschen ermöglicht, wird die Sendeenergie für die Sendespule so hoch gewählt, daß die in der Elektrolyt-Flüssigkeit induzierte Energie die thermische Energie erhöht und dennoch innerhalb der Grenzwerte eines sogenannten zellspezifischen Amplitudenfensters liegt. Die vom Generator erzeugten, gepulsten Ströme umfassen einen Grundstromimpuls von etwa 100 - 1000 Hz, welcher aus der Überlagerung eines Rechteck-Stromes und eines etwa nach einer E-Funktion ansteigenden Stromes, gefolgt von einer wenigstens gleich langen Pulspause, besteht. Die Amplitude der Grundpulsfolge ist mit einer Modulations-Frequenz von 0,5 - 25 Hz in der Amplitude moduliert und die modulierte Grundpulsfolge wird als Pulsfolgeserie für eine Zeitdauer von 0,3 - 1 Sekunde ausgesendet, woran sich jeweils eine Pulsserienpause von 0,7 - 0,5 Sekunden anschließt. Die Patentinhaberin verweist auf die kombinierte Wirkung von Impulsfrequenz, Impulsform, Impulsenergie und Sendespulenform, die es ermöglichen, daß Ionen, insbesondere Protonen aus der intra-korpuralen Elektrolyt-Flüssigkeit, beispielsweise dem Blut, der Lymphe oder dem Liquor gezielt und unmittelbar in die sie umgebenden Gefäßwände und Membranen eingeschleust werden.

In US 48 186 wird eine Vorrichtung zur Verstärkung des Transports eines ausgewählten Ions durch eine bio-molekulare Membran offenbart, welche in einem Raum angeordnet ist, der einem lokalen magnetischen Feld ausgesetzt ist. Das zugeordnete Verfahren umfaßt die Schritte des Erzeugens eines magnetischen Feldes, welches einen nicht verschwindenden zeitlichen Mittelwert aufweist und das Erzeugen eines magnetische Wechselfeldes mit einer Frequenz, die der Zyklotron-Resonanz-Frequenz des vorbestimmten Ions entspricht. Auf Grund des mittels der Zyklotron-Resonanz-Frequenz steuerbaren Energieübertrags auf das vorbestimmte Ion läßt sich der Transport des Ions durch die biomolekulare Membran, beispielsweise eine Zellmembran, steuern. In der europäischen Patentschrift EP 0407006B1 beschreiben die gleichen Erfinder eine Vorrichtung zur Bewegung eines vorgewählten Ions durch eine Membran wobei die Frequenz des magnetischen Wechselfeldes proportional der Zyklotron-Resonanz-Frequenz des vorgewählten Ions ist und, im Unterschied zur obenstehend bezeichneten amerikanischen patentanmeldung, die Proportionalitätskonstante eine vorgewählte, ungerade ganze Zahl größer als eins ist.

Die vorstehend beschriebenen Vorrichtungen, durch welche im Einzelnen die innere Energie der intra-korpuralen Elektrolyt-Flüssigkeiten erhöht oder der Transport vorbestimmter Ionen durch Zellmembranen gesteuert wird, zeigen im Rahmen klinischer Versuche eine gewisse Wirksamkeit.

Es besteht jedoch weiterhin der Bedarf, effektiver auf das Gewebe einzuwirken, und insbesondere den spezifischen Aufbau eines lebenden Organismus bei der Konstruktion einer Vorrichtung zur Magnetfeld-Therapie zu berücksichtigen.

Die auf diese Weise festgelegte Aufgabe der Erfindung wird überraschend einfach mit einer Vorrichtung zur Magnetfeld-Therapie von menschlichem Gewebe nach Anspruch 1 bereitgestellt.

Alle Prozesse im Körper finden auf Zellebene statt. Es muß die Verhaltensweise des Körpers als Ganzes, als geordnete Zellanhäufung, beachtet werden.
* Alle lebenden Organismen werden aus verschiedenen Zellen gebildet.
* Alle Organe werden aus bestimmten Zellenarten gebildet.
* Alle Prozesse im Körper des lebenden Organismus finden auf der Zellebene statt.
* Alle Erkrankungen des lebenden Organismus fangen auf der Zellenebene an und gehen folgende Kette entlang: Zellen → Organe → Organismus.
* Auch Störungen des Ionentransportes finden auf der Zellenebene statt.

Die Magnetfeldtherapie basiert auf der Ausnutzung der statischen und wechselnden Magnetfelder und übt direkten Einfluß auf den lebenden Organismus und biologische Strukturen aus.

Der Frequenzbereich der menschlichen Biorhythmen umfaßt Frequenzen im Bereich von 0 bis 100.000 Hz bzw. 0 bis 1.000.000 Hz.

| | | |
|---|---|---|
| Biorhythmen der Zellen | (generell) | 1000-1.000.000 Hz |
| | (in der Regel) | 1000-100.000 Hz |
| Biorhythmen der Organe | (generell) | 0,1-1000 Hz |
| | (in der Regel) | 0,1-200 Hz |
| | (am häufigsten) | 1-100 Hz |
| Biorhythmen von einzelnen Zellgruppen | | 0-0,5 Hz |

(z.B. Haut), sowie Biorhythmen der einzelnen Organgruppen des Organismus und andere Biorhythmusfrequenzen des Organismus als Ganzes.

Danach zeichnet sich eine derartige Vorrichtung mit einer Einrichtung zur Erzeugung eines magnetischen Feldes mit einer statischen und einer Wechselfeld-Komponente am Ort des zu behandelnden Gewebes dadurch aus, daß das magnetische Wechselfeld eine vorbestimmte Zellenbiorhythmus-Frequenz ν_{z} aufweist und im wesentlichen monochromatisch ist und daß die Vorrichtung ferner eine Einrichtung zur Amplitudenmodulation des magnetischen Wechselfeldes mit einer Modulationsfrequenz νₒ umfaßt, die gleich der Zyklotron-Resonanz-Frequenz B · q / (2π · m) festgelegt ist, wobei B die statische magnetische Flußdichte, q die Ladung und m die Masse des vorbestimmten Ions ist, oder die gleich einer vorbestimmten Organ- oder Zellenbiorhythmus-Frequenz ist.

Somit kann die erfindungsgemäße Vorrichtung zum gleichzeitigen Erhöhen der inneren Energie der Zellen im behandelten Bereich der intrakorpuralen Elektrolyt-Flüssigkeit als auch zum Erhöhen der Energie einer vorbestimmte Gruppe von Ionen verwendet werden, um dem Transport der Ionen im Bereich der Zellmembran zu unterstützen.

Der Hauptgedanke ist die Übertragung der Energie zu den Zellen auf biorhythmischer Frequenz ν_{z}.

Es hat sich herausgestellt, daß nicht nur ein magnetisches Wechselfeld mit der Zyklotronresonanzfrequenz den Transport der vorbestimmten Ionen im Bereich der Zellmembranen erhöhen kann, sondern daß dies auch mit einer Amplitudenmodulation eines magnetischen Wechselfeldes erreicht werden kann, wenn das Wechselfeld mit einer Zellen- oder Organbiorhythmusfrequenz schwingt und die Amplitude mit der Zyklotron-Resonanz-Frequenz moduliert ist. Die Energieübertragung mit den passenden Biorhythmusfrequenzen im behandelten Bereich erfolgt nach folgendem Schema Zelle → Organ → Organgruppe mit gleichzeitiger Wirkung bzw. Hilfe der Zyklotron-Resonanz auf den Ionentransport.

Neben der direkten Einflußnahme auf die Energie eines Ions durch die Einstellung der diesem Ion zugeordneten Zyklotron-Resonanz-Frequenz über die Amplituden-Modulations-Frequenz kann in einem Sekundärprozeß durch die Wechselwirkung dieses Ions mit anderen Ionen auch deren Energie erhöht werden.

Die Vorrichtung spiegelt die Erkenntnis der Erfinder wieder, daß der effektivste Wirkmechanismus in einem lebenden Gewebe sich ausgehend von einzelnen Zellen über die Organe zum Organismus als ganzen fortsetzt. Da spezifischen Zellen eine spezifische Zellbiorhythmus-Frequenz zwischen 1000 Hz und 10⁵ bis 10⁶ Hz zugeordnet werden kann, läßt sich durch die Erzeugung eines magnetischen Wechselfeldes mit einer vorgegebenen Frequenz, die einer bestimmten Zellenbiorhythmus-Frequenz entspricht, definiert auf eine bestimmte Sorte von Zellen einwirken. Diese Einwirkung wird insbesondere auch dadurch festgelegt, daß das Wechselfeld im wesentlichen monochromatisch ist. Eine ungewollte Einwirkung auf andere Frequenzen ist damit minimal. Die im Vergleich zu üblicherweise angewendeten Frequenzen sehr hohe Zellenbiorhythmus-Frequenz von bis zu 10⁵/10⁶ Hz weist weiterhin den Vorteil auf, daß trotz niedriger Feldamplitude sehr hohe Energien an die Zellen übertragen werden können. Diese Energieabgabe, welche über das durch das hochfrequente magnetische Wechselfeld erzeugte elektrische Feld gleicher Frequenz bedingt sein kann, verläuft resonant für die mit der Zellenbiorhythmus-Frequenz ausgewählten Zellen.

Mit der erfindungsgemäßen Vorrichtung läßt sich eine umfassendere und effektivere Magnetfeldtherapie als mit herkömmlichen Vorrichtungen durchführen, beispielsweise auf dem Gebiet der regressiven Störungen, Entzündungen, Kreislaufstörungen, Frakturen und Traumata etc.

Liegt die über die Organbiorhythmus-Frequenz eingestellte Amplituden-Modulations-Frequenz zwischen 0,1 Hz - 1000 Hz so ist sichergestellt, daß entsprechend der Erkenntnis der Erfinder für ein optimales Einwirken auf das zu behandelnde menschliche Gewebe, die Energieabgabe bei der Zellenbiorhythmus-Frequenz um mehrere Größenordnungen höher liegt, als die Energieabgabe bei der Organbiorhythmus-Frequenz (die übertragene Leistung bei fester Amplitude ist proportional zur dritten Potenz der Frequenz). Zur Maximierung der Energieabgabe aufgrund der Resonanzbedingung für die Zellen, die Organe bzw. die Ionen kann die Einrichtung zur Amplituden-Modulation das magnetische Wechselfeld im wesentlichen sinusförmig modulieren, um maximale Monochromazität zu erreichen, wobei auch andere Anregungsformen möglich sind (Rechteck, Dreieck, Trapez, Sägezahn).

Um das durch die Erfinder gefundene Wirkprinzip, ausgehend von der zu behandelnden Zelle ν_{z} und weiterführend über das zu behandelnde Organ νₒ zum gesamten Organismus zu vervollständigen, kann die Vorrichtung ferner eine Einrichtung umfassen, welche zur Amplituden-Modulation des mit νₒ amplitudenmodulierten magnetischen Wechselfeldes mit einer Frequenz dient, die gleich einer vorbestimmten Organbiorhythmus-Frequenz ν_{R} ist. Diese Organismusbiorhythmus-Frequenz liegt im allgemeinen zwischen 0 - 1.000 Hz und stellt somit entsprechend dem gefundenen Prinzip sicher, daß die bei der Organismusbiorhythmus-Frequenz an das Gewebe abgegebene Energie sehr viel geringer ist als die Energie, welche bei der Organbiorhythmus-Frequenz vom Gewebe absorbiert wird.

Für die Erzeugung des magnetischen Wechselfeldes und/oder des statischen magnetischen Feldes können alle herkömmlichen Vorrichtungen verwendet werden. Vorteilhafterweise lassen sich zwei voneinander beabstandete Teilspulen mit zueinander ausgerichteten Längsachsen verwenden, um den zwischen den Teilspulen leicht zugänglichen Raum als Behandlungsraum zu definieren.

Um eine bestimmte Therapieform zu ermöglichen, können die Einrichtungen zur Erzeugung der Felder derart angeordnet sein, daß der Feldvektor des zeitlich konstanten magnetischen Feldes im wesentlichen mit der Richtung des Feldvektors des magnetischen Wechselfeldes zusammenfällt. Damit wird erreicht, daß gleichzeitig in einem bestimmten Frequenzintervall verschiedene Ionen mit ihrer ZyklotronFrequenz angeregt werden können. Um eine andere Therapieform zu ermöglichen, können die Einrichtungen zur Erzeugung der magnetischen Felder auch derart angeordnet, daß die Richtung des Feldvektors des zeitig konstanten magnetischen Feldes im wesentlichen senkrecht zur Richtung des Feldvektors des magnetischen Wechselfeldes liegt. Hierdurch wird erreicht, daß im wesentlichen nur ein Ion bzw. nur ein Organ des zu behandelnden Gewebes mit zugeordneter Energie versorgt wird.

Die Vorrichtung kann je nach Bedarf mit einer Vielzahl von Einrichtungen zur Erzeugung von magnetischen Wechselfeldern und statischen magnetischen Feldern in beliebiger Richtungen ausgestattet werden. Vorteilhafterweise umfaßt die Vorrichtung demnach Mittel zur Erzeugung eines magnetischen Wechselfeldes in drei zu einander senkrecht liegenden Raumrichtungen. Weiterhin können Mittel zur Erzeugung eines statischen magnetischen Feldes in drei zueinander senkrecht liegenden Raumrichtungen umfaßt sein. Auf diese Weise lassen sich entsprechende Prozesse in beliebige Richtungen hervorrufen, deren Wirkung sich durch die hervorgerufene Energieaufnahme im Gewebe addieren. Um den Einfluß des Erdmagnetfeldes, insbesondere bei der Anwendung von niedrigen Magnetfeldstärken zu berücksichtigen, kann die Vorrichtung Mittel zur Richtungsabhängigen Erfassung des am Ort des zu behandelnden Gewebes vorhandenen Erdmagnetfeldes und zum Berücksichtigen des erfaßten Erdmagnetfeldes für das Einstellen des statischen magnetischen Feldes und/oder des magnetischen Wechselfeldes umfassen. Diese vor der Anwendung durchgeführte Justage der Vorrichtung auf das vorhandene Erdmagnetfeld, oder eine andere statische oder Wechselfeld-Störung wird dann während der Anwendung beibehalten. Somit ist eine Verfälschung der einer Ionenart zugeordneten Zyklotron-Resonanz-Frequenz oder einem Organ zugeordneten Organbiorhythmusfrequenz ausgeschlossen.

Die Vorrichtung zur Erzeugung des statischen Magnetfeldes kann derart eingestellt sein, daß das statische Magnetfeld am Ort des zu behandelnden Gewebes zwischen 10⁻⁵ Tesla und 0,1 Tesla beträgt. Diese niedrige Flußdichte ermöglicht eine Therapie, welche natürlichen Flußdichten, die vor allem durch das Erdmagnetfeld hervorgerufen werden, entsprechen. Somit können beispielsweise Träger eines Herzschrittmachers einer Therapie mit der erfindungsgemäßen Vorrichtung ohne Gefahr unterzogen werden. Dies trifft insbesondere dann zu, wenn die Amplitude des magnetischen Wechselfeldes auch zwischen 10⁻⁵ Tesla und 5· 10⁻³ Tesla, wobei der Standardanwendungsbereich vorzugsweise im Bereich von 1· 10⁻⁴ beträgt.

Um die erfindungsgemäße Vorrichtung auf die jeweiligen Bedürfnisse flexibel einstellen zu können, kann die Vorrichtung über eine Eingabe/Ausgabevorrichtung, einen Kartenleser, wie insbesondere einen Magnetkartenleser oder andere Kommunikationseinrichtungen, wie beispielsweise Chipkartenleser oder Leser für optische Datenträger aufweisen, über den Parameter zum Einstellen der Vorrichtung eingelesen werden können. Zum Einstellen der Vorrichtung kann dieser Kartenleser über einen Controller an eine Registereinrichtung angeschlossen sein, die zusammen mit zumindest einem Generator an eine Modulationseinheit zur Erzeugung eines Signals zum Ansteuern der zumindest einen Spule verbunden sein, die das magnetische Wechselfeld erzeugt. Um eine möglichst phasensynchrone und damit ein gleichgerichtetes Einwirken auf das Gewebe sicherzustellen, können einander zugeordnete Spulen, beispielsweise die obenstehend bezeichneten Teilspulen an eine Synchronisationeinrichtung angeschlossen sein, welche den wenigstens einen Generator entsprechend steuert. Um die Spule zur Erzeugung eines konstanten Magnetfeldes anzusteuern, kann die Registereinrichtung ferner an eine Strom- oder Spannungsquelle angeschlossen sein.

Die Erfindung wird im folgenden durch das Beschreiben verschiedener Ausführungsformen unter Berücksichtigung der anliegenden Zeichnungen erläutert. Es zeigen
- Fig. 1: schematisch eine Einrichtung einer erfindungsgemäßen Vorrichtung zur Erzeugung eines magnetischen Wechselfeldes und eines statischen Magnetfeldes;
- Fig. 2: schematisch den zeitlichen Verlauf des Magnetfeldes für die in Fig. 1 gezeigter Einrichtung wobei
- Fig. 2a: das mit der Amplituden-Modulations-Frequenz νₒ modulierte magnetische Wechselfeld mit der Zellenbiorhythmus-Frequenz ν_{z} darstellt; und
- Fig. 2b: das magnetische Wechselfeld der Fig. 2a darstellt, wobei dieses ferner mit der Organismusbiorhythmus-Frequenz ν_{R} amplituden-moduliert ist; und
- Fig. 3: ein Blockschaltbild einer beispielhaften Ausführungsform der Erfindung.

Die Vorrichtung zur Magnetfeld-Therapie von menschlichem Gewebe, welche zumindest eine Einrichtung zur Erzeugung eines magnetischen Feldes mit einer statischen Komponente und einer Wechselfeldkomponente am Ort des zu behandelnden Gewebes umfaßt, zeichnet sich erfindungsgemäß dadurch aus, daß das magnetische Wechselfeld monochromatisch mit einer vorbestimmten Zellenbiorhythmus-Frequenz ν_{z} ist. Die erfindungsgemäße Vorrichtung weist ferner eine Einrichtung zur Amplituden-Modulation des vorgeschriebenen magnetischen Wechselfeldes mit einer Modulations-Frequenz ν₀, welche entweder gleich der Zyklotronfrequenz eines vorbestimmten Ions im Gewebe oder eine vorbestimmte Organbiorhythmus-Frequenz ist. Die Verwendung der erfindungsgemäßen Vorrichtung umfaßt die Schritte:
1. Auswahl der zu behandelnden Zellen, beispielsweise durch Anamnese, wobei die Bestimmung der zu behandelnden Zellenart die einzustellende Zellenbiorhythmus-Frequenz ν_{z} festlegt.
2. In Abhängigkeit der anzuwendenen Therapie, Wahl der Richtung und Stärke des magnetischen Wechselfeldes und des statischen Magnetfeldes.
3. Wahl der Modulationsfrequenz νₒ zur Amplituden-Modulation des magnetischen Wechselfeldes wobei je nach Therapieform νₒ die Zyklotronfrequenz einer vorbestimmten Ionenart im zubehandelnden Gewebe oder eine vorbestimmte Organbiorhythmus-Frequenz ist.
4. Einstellen der vorbestimmten Parameter der Vorrichtung.

In einer Ausführungsform der Erfindung liegt die vorbestimmte und einstellbare Zellenbiorhythmus-Frequenz ν_{z} zwischen 1000 und 10⁶ Hz. Dieses magnetische Feld mit der Zellenbiorhythmus-Frequenz ν_{z} ruft im zu behandelnden Gewebe unter der Annahme einer willkürlich genommenen Schleife eine Energieaufnahme durch die Induzierung eines Stromes hervor. Die im Gewebe aufgenommene Leistung ist proportional zur Quadrat des Produktes der Magnetfeld-Amplitude und der Frequenz.

In einer Ausführungsform der Erfindung wird die Amplituden-Modulations-Frequenz νₒ in der Vorrichtung derart eingestellt, daß sie durch den Ausdruck νₒ = B · q / 2π · m bestimmt ist, wobei B die statische magnetische Flußdichte, q die Ladung und m die Masse des vorbestimmten Ions ist, dessen Energie im Rahmen der Anwendung des magnetischen Feldes erhöht werden soll. Zum Zwecke einer weiteren Therapieform wird die Amplituden-Modulation-Frequenz in einer spezifischen Ausführungsform der erfindungsgemäßen Vorrichtung auf eine Organbiorhythmus-Frequenz zwischen 0,1 Hz und 1000 Hz eingestellt. Durch die Einrichtungen zu Amplituden-Modulation wird das magnetische Wechselfeld im wesentlichen sinusförmig moduliert.

In einer Ausgestaltung der Erfindung umfaßt die Vorrichtung ferner eine Einrichtung zur Amplituden-Modulation des mit νₒ amplituden-modulierten magnetischen Wechselfeldes. Diese zweite Amplituden-Modulation wird durch die genannte Einrichtung mit einer vorbestimmten Organismusbiorhythmus-Frequenz ν_{R} durchgeführt.

Optional kann das entstehende Signal nach dem gefundenen Wirkprinzip nochmals mit einer sogenannten Frequenz der Grenzkurve amplitudenmoduliert werden.

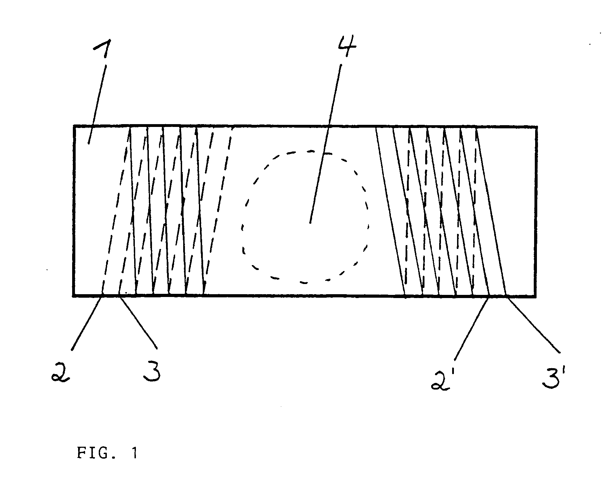

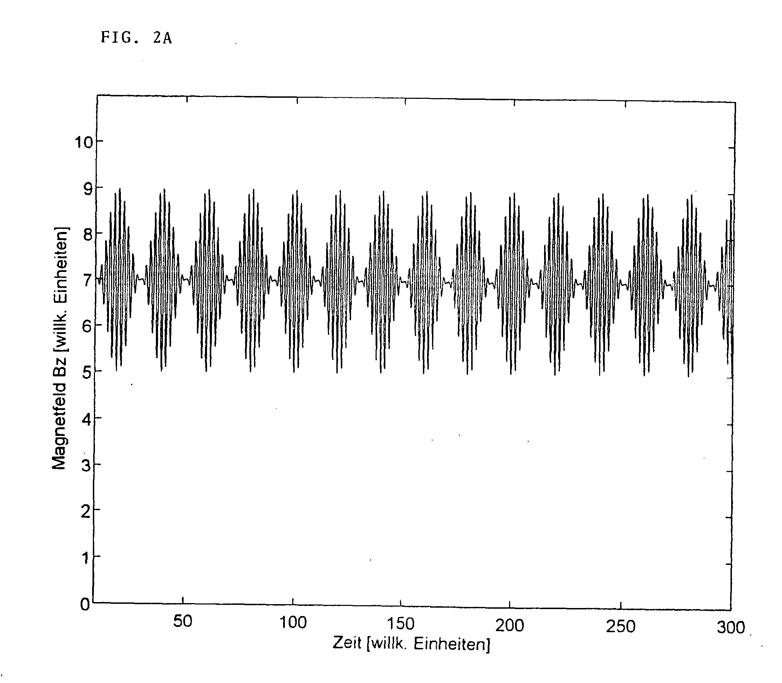

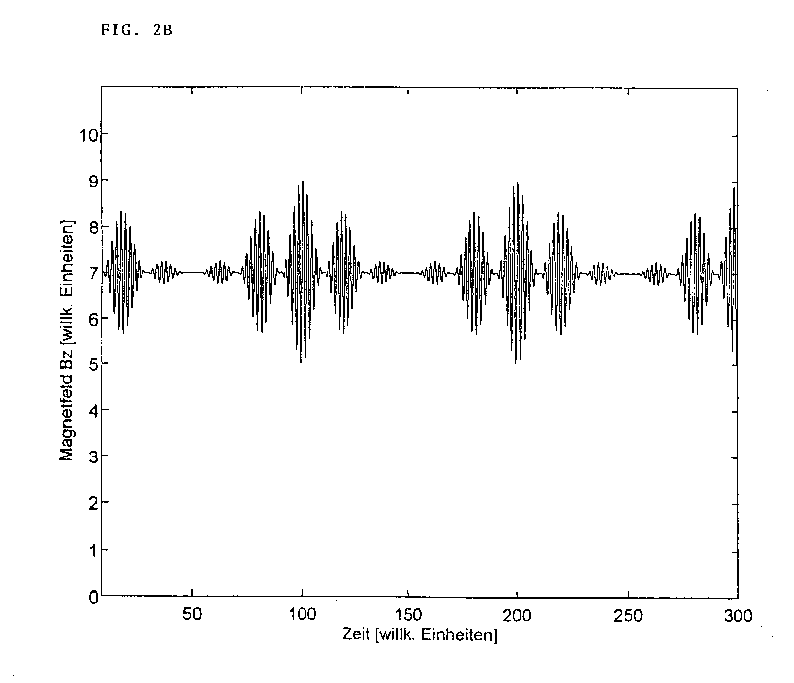

Die Einrichtungen zur Erzeugung des magnetischen Wechselfeldes und/oder des statischen magnetischen Feldes können je nach Ausgestaltung der Erfindung alle herkömmlichen Mittel zur Erzeugung von Magnetfeldern umfassen. Fig. 1 zeigt eine derartige beispielhafte Ausführungsform wobei jeweils zur Erzeugung des magnetischen Wechselfeldes und zur Erzeugung des statischen magnetischen Feldes zwei voneinander beabstandete Teilspulen 2,2' bzw. 3,3' auf einem Spulenkörper 1 angeordnet sind. Die benachbarten Spulen 2,3 bzw. 2',3' sind dabei voneinander isoliert und werden elektrisch getrennt angesteuert. Der Bereich 4 zwischen den Teilspulen definiert im wesentlichen den Behandlungsraum, in welchem das zu behandelnde Gewebe, beispielsweise ein in Form eines Körperteils, eingebracht wird. Zu diesem Zweck kann der Spulenkörper 1 eine in Fig. 1 nicht gezeigte seitliche Öffnung aufweisen. Weiterhin kann die Einführung auch entlang der Längsachse des Spulenkörpers durchgeführt werden. Die Richtung des Feldvektors des zeitlich konstanten magnetischen Feldes liegt für die in Fig. 1 dargestellte beispielhafte Ausgestaltung der Erfindung parallel zum Feldvektor des magnetischen Wechselfeldes. Das resultierende magnetische Feld im Bereich 4 ist in den Fig. 2a und 2b schematisch dargestellt, wobei die Z-Achse in Längsrichtung zum Spulenkörper angenommen wird. Fig. 2a zeigt das resultierende Magnetfeld Bz im Z-Richtung in Abhängigkeit der Zeit für ein monochromatisches magnetisches Wechselfeld, welches eine vorbestimmte Zellenbiorhythmus-Frequenz aufweist und das mit einer Amplituden-Modulations-Frequenz moduliert ist, wobei die Modulations-Frequenz sehr viel kleiner als die Zellenbiorhythmus-Frequenz ist. Fig 2b zeigt den Verlauf des magnetischen Feldes in Z-Richtung in Abhängigkeit der Zeit für den Fall, daß zusätzlich zur Amplituden-Modulation mit der Frequenz νₒ, das resultierende Magnetfeld wiederum amplituden-moduliert wird, nun mit der Organismusbiorhythmus-Frequenz ν_{R}, die maximal 0,5 Hz beträgt. Fig. 2a stellt somit eine Schwingung dar, welche sich aus drei Anteile zusammensetzt: aus der Grundschwingung mit der Trägerfrequenz ν_{Z} und Schwingungen der beiden Seitenbänder mit ν_{Z} - ν_{O} und ν_{Z} + ν_{O}.

Ausführungsformen der Erfindung sind jedoch nicht auf Fälle beschränkt, bei welchen die Feldvektoren des zeitlich konstanten magnetischen Feldes und des statischen magnetischen Feldes zusammen fallen. In einer Reihe von Ausführungsformen der Erfindung sind die Spulen zur Erzeugung der magnetischen Felder derart angeordnet, daß der Feldvektor des statischen magnetischen Feldes senkrecht zum Feldvektor des magnetischen Wechselfeldes steht.

Am flexibelsten an die jeweiligen Bedürfnisse anpassbar ist eine Ausführungsform der Erfindung, welche Mittel zur Erzeugung eines magnetischen Wechselfeldes in drei zueinander senkrecht liegenden Raumrichtungen und Mittel zur Erzeugung eines statischen magnetischen Feldes in drei senkrecht zueinander liegenden Raumrichtungen umfaßt. In diesem Fall sind insgesamt vorzugsweise 15 Spulen, welche jeweils zwei Teilspulen umfassen in geeigneter Weise auf einem Spulenkörper angebracht und verschaltet sind, sodaß zwischen den Teilspulen sich wieder der Behandlungsraum ergibt. Durch geeignete und ausgewählte Anregungen der Spulen lassen sich eine Vielzahl von Magnetfeldkonfigurationen erzeugen. Neben der schon beschriebenen Anordnung, bei welcher ein statisches und ein magnetisches Wechselfeld in Z-Richtung erzeugt werden, wobei das magnetische Wechselfeld beispielsweise mit der Zyklotron-Resonanz-Frequenz, die vom statischen Magnetfeld abhängt, moduliert ist. Statt des Wechselfeldes in Z-Richtung kann jedoch auch ein Wechselfeld in Y-Richtung, in X-Richtung, oder gleichzeitig in X- und Y-Richtung angewendet werden. Die Frequenz der jeweiligen Felder ist auf die ausgewählte Zellenbiorhythmus-Frequenz eingestellt. In gleicher Weise sind die Wechselfelder in den jeweiligen Richtungen mit der Zyklotron-Resonanz-Frequenz oder mit der Organbiorhythmus-Frequenz moduliert. Weiterhin ist in einer Konfiguration auch möglich, ein statisches Feld in Z-Richtung einzustellen und ein magnetisches Wechselfeld mit den zugeordneten Amplituden-Modulationen in alle drei Raumrichtungen X, Y, Z anzuregen. Im allgemeinsten Fall werden sowohl statische Magnetfelder als auch amplitudenmodulierte magnetische Wechselfelder in alle 3 Raumrichtungen erzeugt.

Durch die erfindungsgemäße Vorrichtung werden statische magnetische Felder mit einer Flußdichte mit 10⁻⁵ Tesla bis 0,1 Tesla erzeugt. Bei einer Flußdichte von 10⁻⁴ Tesla ergibt sich damit beispielsweise für ein H⁺-Zyklotron-Resonanz-Frequenz von 1528 Hz, für Li⁺ 218,3 Hz, während die Frequenzen für schwerere Ionen sehr viel niedriger liegen, beispielsweise K⁺ 38,4 Hz und Cu⁺ 24 Hz.

| Ionen, chemische Elemente | Frequenzen (Hz) für | Frequenzen (Hz) für |
|---|---|---|
| | B=1·10⁻⁴ T;1 G | B=(0,2-07)·10⁻⁴ T |
| H(1)⁺ | 1528 | 305,6-1070 |
| O(16)²⁻ | 191 | 38,2-133,7 |
| Na(23)⁺ | 66,4 | 13,3-46,5 |
| P(31)³⁺ P(31)⁵⁻ | 147,9 246,5 | 29,4-103 49,3-172,6 |
| K(40)⁺ | 38,4 | 7,68-26,91 |
| Mn(55)²⁺ | 55, 6 | 11,2-38,92 |
| Fe(56)³⁺ | 81,19 | 16,38-57,33 |
| Fe(56)²⁺ | 54,6 | 10,92-38,22 |
| Cu(64)⁺ | 24 | 4,8-16,8 |
| Se(79)²⁻ | 38,8 | 7,76-27,16 |
| Br(80)⁻ | 19,1 | 3,82-13,4 |
| Ag(108)⁺ | 14 | 2,8-9,9 |
| J(126)⁻ | 12,13 | 2,4-8,5 |
| Au(197)⁺ | 7,8 | 1,56-5,5 |

Diese Tabelle zeigt die Möglichkeit der gleichzeitigen Einwirkung auf die Organe und Ionen im Organbiorhythmusbereich 1-200 Hz bei B₌=0,2-1 G und auf Protronen im Frequenzbereich 300-1530 Hz bei B₋=0,2-1 G.

Mit den auf diese Weise berechneten Zyklotron-ResonanzFrequenzen wird, wie obenstehend erläutert, das magnetische Wechselfeld, welches die Zellenbiorhythmus-Frequenz aufweist, amplituden-moduliert. Die Amplitude des magnetischen Wechselfeldes ist wie das statische Magnetfeld zwischen 10⁻⁵ Tesla und 7· 10⁻⁴ Tesla einstellbar.

Je nach Anwendung kann bei Bedarf die Amplitude jedoch größer sein.

In einer Ausgestaltung umfaßt die Vorrichtung eine Einrichtung zur richtungsabhängigen Erfassung des am Ort des zu behandelnden Gewebes vorhandenen Magnetfeldes, beispielsweise ein Magnetometer. Das erfasste Magnetfeld wird nun zum Einstellen des in Abhängigkeit der gewählten Therapie ausgewählten statischen Magnetfeldes vor der Behandlung berücksichtigt. Soll beispielsweise in Z-Richtung ein statisches Magnetfeld von 10⁻⁴ Tesla angewendet werden, das Erdmagnetfeld jedoch in diese Richtung 5 · 10⁻⁶ Tesla beträgt, so wird die entsprechende Spule derart angesprochen, daß sie ein statisches Magnetfeld von 1,5·10⁻⁵ Tesla mit umgekehrtem oder 1,5·10⁻⁵ Tesla mit den direkten Vorzeichen erzeugt. In gleicher Weise werden die Komponenten des Erdmagnetfeldes in X- und Y-Richtung berücksichtigt oder kompensiert und während des Betriebs konstant gehalten.

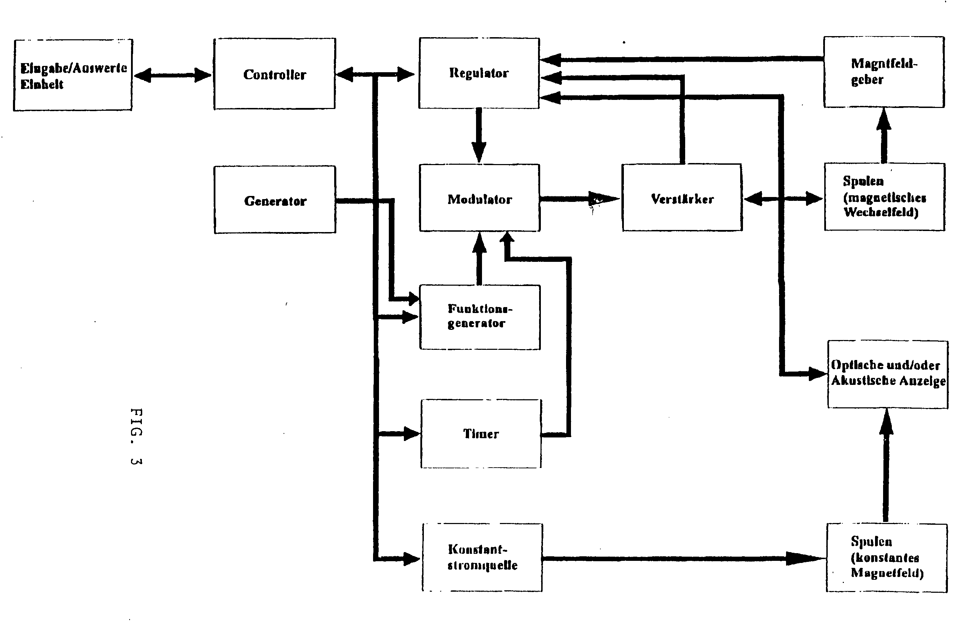

In Fig. 3 ist der Aufbau einer beispielhaften Ausführungsform in der Erfindung in einem Blockschaltbild erläutert. Danach weist die Vorrichtung einen Kartenleser auf, über den Parameter zum Einstellen der Vorrichtung eingelesen werden. Dies betrifft beispielsweise Angaben über die Zellenbiorhythmus-Frequenz, die Zyklotron-Resonanz-Frequenz bzw. die Organbiorhythmus-Frequenz, die Organismus- oder Zellenbiorhythmusfrequenz und die Flußdichten der zu erzeugenden einzelnen Magnetfelder in die verschiedenen Richtungen. Der Kartenleser ist über einen Controller an eine Registereinrichtung eingeschlossen, in der die eingelesenen Parameter gespeichert werden. Die Registereinrichtung ist zusammen mit dem Generator bzw. den Generatoren an eine Modulationseinheit zur Erzeugung eines Signals zum Ansteuern der Spule, welche das magnetische Wechselfeld erzeugt, verbunden. Sind mehrere Spulen zur Erzeugung eines wechselnden Magnetfeldes umfaßt, so kann die erfindungsgemäße Vorrichtung auch mehrere, den jeweiligen Spulen zugeordnete Modulationseinheiten aufweisen. Um eine phasengleiche Ansteuerung der einzelnen Spulen zum Erzeugen eines wechselnden Magnetfeldes sicherzustellen, sind die Spulen über eine Synchronisationseinheit mit dem Generator bzw. den Generatoren verbunden. Die Vorrichtung nach Fig. 3 umfaßt weiterhin eine Spannungsquelle, welche äquivalent auch durch eine Stromquelle ausgeführt sein kann, die über das Register angesteuert wird und eine konstante Spannung bzw. einen konstanten Strom zur Erregung der Spule zum Erzeugen eines statischen Magnetfeldes bereitstellt.

Zur Erzeugung der monochromatischen Schwingungen mit der Zellenbiorhythmus-Frequenz wird je nach Ausgestaltung der Erfindung eine Spannungsresonanz (Reihenschaltung einer Induktivität und einer Kapazität) oder eine Stromresonanz (Parallelschaltung einer Induktivität mit einer Kapazität) in der entsprechenden Einrichtung ausgenutzt. Dieser LC-Kreis wird dann jeweils auf die vorbestimmte Zellenbiorhythmus-Frequenz abgestimmt. Die Erzeugung der Modulations-Frequenzen, d.h. der Organbiorhythmus-Frequenz, der Zyklotron-Resonanz-Frequenz bzw. der Organismusbiorhythmus-Frequenz wird in zugeordneten LC-Kreisen auf gleiche Weise hergestellt.

## Patentansprüche

1. Vorrichtung zur Magnetfeldtherapie von menschlichem Gewebe, welche zumindest eine Einrichtung zur Erzeugung eines magnetischen Feldes mit einer statischen und einer Wechselfeldkomponente am Ort des zu behandelnden Gewebes umfaßt,
**dadurch gekennzeichnet, daß**
das magnetische Wechselfeld eine vorbestimmte Zellenbiorhythmusfrequenz ν_{z} zwischen 1000 Hz und 10⁶ Hz aufweist und im wesentlichen monochromatisch ist und die Vorrichtung ferner eine Einrichtung zur Amplitudenmodulation des magnetischen Wechselfeldes mit einer Modulationsfrequenz νₒ umfaßt, die eine vorbestimmte Organbiorhythmusfrequenz zwischen 0,1 Hz und
1000 Hz ist, bei welcher die Einrichtung zur Amplitudenmodulation das magnetische Wechselfeld im wesentlichen sinusförmig moduliert.

2. Vorrichtung nach Anspruch 1,
**dadurch gekennzeichnet, daß**
die Vorrichtung ferner eine Einrichtung umfaßt zur Amplitudenmodulation des mit νₒ amplitudenmodulierten magnetischen Wechselfeldes mit einer Frequenz, die gleich einer vorbestimmten Organismusbiorhythmusfrequenz ν_{R} ≤ 0,5 Hz ist.

3. Vorrichtung nach einem der vorstehenden Ansprüche,
**dadurch gekennzeichnet, daß**
eine Einrichtung zur Erzeugung des magnetischen Wechselfeldes und/oder des statischen magnetischen Feldes zwei voneinander beabstandete Teilspulen mit zueinander ausgerichteten Längsachsen umfaßt.

4. Vorrichtung nach einem der vorstehenden Ansprüche,
**dadurch gekennzeichnet, daß**
die Richtung des Feldvektors des zeitlich konstanten magnetischen Feldes zumindest am Ort des zu behandelnden Gewebes im wesentlichen mit der Richtung des Feldvektors des magnetischen Wechselfeldes zusammenfällt.

5. Vorrichtung nach einem der vorstehenden Ansprüche,
**dadurch gekennzeichnet, daß**
die Richtung des Feldvektors des zeitlich konstanten magnetischen Feldes zumindest am Ort des zu behandelnden Gewebes im wesentlichen senkrecht zur Richtung des Feldvektors des magnetischen Wechselfeldes liegt.

6. Vorrichtung nach einem der vorstehenden Ansprüche,
**dadurch gekennzeichnet, daß**
Mittel zur Erzeugung eines magnetischen Wechselfeldes in drei senkrecht zueinander liegenden Raumrichtungen umfaßt sind.

7. Vorrichtung nach einem der vorstehenden Ansprüche,
**dadurch gekennzeichnet, daß**
Mittel zur Erzeugung eines statischen magnetischen Feldes in drei senkrecht zueinander liegenden Raumrichtungen umfaßt sind.

8. Vorrichtung nach einem der vorstehenden Ansprüche,
**dadurch gekennzeichnet, daß**
die Vorrichtung Mittel zur richtungsabhängigen Erfassung eines am Ort des zu behandelnden Gewebes vorhandenen Erdmagnetfeldes und zum Berücksichten des erfaßten Erdmagnetfeldes für das Einstellen des statischen magnetischen Feldes vor der Erzeugung des statischen magnetischen Feldes und/oder des magnetischen Wechselfeldes umfaßt.

9. Vorrichtung nach einem der vorstehenden Ansprüche,
**dadurch gekennzeichnet, daß**
das statische Magnetfeld zwischen 10⁻⁵ Tesla und 7·10⁻⁴ Tesla beträgt.

10. Vorrichtung nach einem der vorstehenden Ansprüche,
**dadurch gekennzeichnet, daß**
die Amplitude des magnetischen Wechselfeldes zwischen 10⁻⁵ Tesla und 7·10⁻⁴ Tesla beträgt.

11. Vorrichtung nach einem der vorstehenden Ansprüche,
**dadurch gekennzeichnet, daß**
die Vorrichtung einen Kartenleser aufweist, über welchen Parameter zum Einstellen der Vorrichtung eingelesen werden.

12. Vorrichtung nach Anspruch 11,
**dadurch gekennzeichnet, daß**
der Kartenleser über einen Controller an eine Registereinrichtung angeschlossen ist, die zusammen mit zumindest einem Generator an eine Modulationseinheit zur Erzeugung eines Signals zum Ansteuern der zumindest einen Spule angeschlossen ist, welche das magnetische Wechselfeld erzeugt.

13. Vorrichtung nach Anspruch 12,
**dadurch gekennzeichnet, daß**
einander zugeordnete Spulen an eine Synchronisationseinrichtung angeschlossen sind, welche den wenigstens einem Generator derartig steuert, das die Spulen im wesentlichen phasengleich angesteuert werden.

14. Vorrichtung nach Anspruch 13,
**dadurch gekennzeichnet, daß**
die Registereinrichtung ferner an eine Spannungsquelle angeschlossen ist, welche zumindest eine Spule zur Erzeugung eines konstanten Magnetfeldes ansteuert.

## Claims

1. Device for magnetic field therapy of human tissue which comprises at least one device for generating a magnetic field with a static and an alternating field component at the site of the tissue to be treated,
**characterised in that**
the alternating magnetic field has a predetermined cell biorhythm frequency v_{z} between 1000 Hz and 10⁶ Hz and is essentially monochromatic and the device further comprises a device for amplitude modulation of the alternating magnetic field with a modulation frequency vₒ which is a predetermined organ biorhythm frequency between 0.1 Hz and 1000 Hz, whereby the device for amplitude modulation modulates the alternating magnetic field essentially sinusoidally.

2. Device according to claim 1,
**characterised in that**
the device further comprises a device for amplitude modulation of the alternating magnetic field amplitude-modulated with vₒ with a frequency which is equal to a predetermined organism biorhythm frequency of v_{R} ≤ 0.5 Hz.

3. Device according to one of the preceding claims,
**characterised in that**
a device for generating the alternating magnetic field and / or the static magnetic field comprises two coil sections spaced apart from each other with longitudinal axes orientated relative to each other.

4. Device according to one of the preceding claims,
**characterised in that**
the direction of the field vector of the temporally constant magnetic field coincides at least at the site of the tissue to be treated essentially with the direction of the field vector of the magnetic alternating field.

5. Device according to one of the preceding claims,
**characterised in that**
the direction of the field vector of the temporally constant magnetic field lies, at least at the site of the tissue to be treated, essentially perpendicular to the direction of the field vector of the magnetic alternating field.

6. Device according to one of the preceding claims,
**characterised in that**
means for generating an alternating magnetic field are contained in three spatial directions lying perpendicular to each other.

7. Device according to one of the preceding claims,
**characterised in that**
means for generating a static magnetic field are contained in three spatial directions lying perpendicular to each other.

8. Device according to one of the preceding claims,
**characterised in that**
the device comprises means for direction-dependent detection of the earth's magnetic field present at the site of the tissue to be treated and for consideration of the detected earth's magnetic field for the setting of the static magnetic field before the generation of the static magnetic field and / or the magnetic alternating field.

9. Device according to one of the preceding claims,
**characterised in that**
the static magnetic field is between 10⁻⁵ Tesla and 7-10⁻⁴ Tesla.

10. Device according to one of the preceding claims,
**characterised in that**
the amplitude of the alternating magnetic field is between 10⁻⁵ Tesla and 7-10⁻⁴ Tesla.

11. Device according to one of the preceding claims,
**characterised in that**
the device comprises a card reader, via which parameters are read for the setting of the device.

12. Device according to claim 11,
**characterised in that**
the card reader is connected via a controller to a register device which is connected together with at least one generator to a modulation unit for generating a signal for controlling the at least one coil which generates the magnetic alternating field.

13. Device according to claim 12,
**characterised in that**
coils assigned to each other are connected to a synchronisation device which controls the at least one generator in such a way that the coils are controlled essentially in phase.

14. Device according to claim 13,
**characterised in that**
the register device is further connected to a voltage source which controls at least one coil for generating a constant magnetic field.

## Revendications

1. Dispositif pour la thérapie par champ magnétique de tissu humain, qui comprend au moins un dispositif pour générer un champ magnétique avec une composante statique et une composante de champ alternatif à l'emplacement du tissu à traiter,
**caractérisé en ce que**
le champ alternatif magnétique présente une fréquence de biorythme de cellule vₛ prédéfinie comprise entre 1000 Hz et 10⁶ Hz et est sensiblement monochromatique et le dispositif comprend également un appareil pour la modulation d'amplitude du champ alternatif magnétique avec une fréquence de modulation vₒ, qui est une fréquence de biorythme d'organe prédéfinie comprise entre 0,1 Hz et 1000 Hz, à laquelle l'appareil pour la modulation d'amplitude module le champ alternatif magnétique sensiblement en forme de sinusoïde.

2. Dispositif selon la revendication 1,
**caractérisé en ce que**
le dispositif comprend également un appareil pour la modulation d'amplitude du champ alternatif magnétique modulé en amplitude avec vₒ avec une fréquence qui est égale à une fréquence de biorythme d'organisme prédéfinie (v_{R} ≤ 0,5 Hz).

3. Dispositif selon l'une quelconque des revendications précédentes, **caractérisé en ce que**
un appareil pour générer le champ alternatif magnétique et/ou le champ magnétique statique comprend deux bobines partielles espacées l'une de l'autre avec des axes longitudinaux orientés l'un vers l'autre.

4. Dispositif selon l'une quelconque des revendications précédentes,
**caractérisé en ce que**
la direction du vecteur du champ magnétique constant dans le temps coïncide au moins à l'emplacement du tissu à traiter sensiblement avec la direction du vecteur de champ du champ alternatif magnétique.

5. Dispositif selon l'une quelconque des revendications précédentes,
**caractérisé en ce que**
la direction du vecteur de champ du champ magnétique constant dans le temps se situe au moins à l'emplacement du tissu à traiter sensiblement perpendiculairement à la direction du vecteur de champ du champ alternatif magnétique.

6. Dispositif selon l'une quelconque des revendications précédentes,
**caractérisé en ce que**
des moyens destinés à générer un champ alternatif magnétique sont inclus dans trois directions d'espace disposées perpendiculairement entre elles.

7. Dispositif selon l'une quelconque des revendications précédentes,
**caractérisé en ce que**
des moyens destinés à générer un champ magnétique statique sont inclus dans trois directions d'espace perpendiculaires entre elles.

8. Dispositif selon l'une quelconque des revendications précédentes,
**caractérisé en ce que**
le dispositif comprend des moyens pour l'enregistrement dépendant de la direction d'un champ magnétique terrestre présent à l'emplacement du tissu à traiter et pour la prise en compte du champ magnétique terrestre détecté pour le réglage du champ magnétique statique avant la génération du champ magnétique statique et/ou du champ alternatif magnétique.

9. Dispositif selon l'une quelconque des revendications précédentes,
**caractérisé en ce que**
le champ magnétique statique est conclu entre 10⁻⁵ teslas et 7.10⁻⁴ teslas.

10. Dispositif selon l'une quelconque des revendications précédentes,
**caractérisé en ce que** l'amplitude du champ alternatif magnétique est comprise entre 10⁻⁵ teslas et 7.10⁻⁴ teslas.

11. Dispositif selon l'une quelconque des revendications précédentes,
**caractérisé en ce que**
le dispositif présente un lecteur de cartes par lequel des paramètres pour le réglage du dispositif sont entrés.

12. Dispositif selon la revendication 11,
**caractérisé en ce que**
le lecteur de cartes est raccordé par un contrôleur à un dispositif de registre qui est raccordé en même temps qu'au moins un générateur à une unité de modulation pour générer un signal pour l'activation de la au moins une bobine, qui génère le champ alternatif magnétique.

13. Dispositif selon la revendication 12,
**caractérisé en ce que**
des bobines attribuées entre elles sont raccordées à un dispositif de synchronisation qui commande le au moins un générateur de telle sorte que les bobines sont activées sensiblement en coïncidence de phase.

14. Dispositif selon la revendication 13,
**caractérisé en ce que**
le dispositif de registre est raccordé également à une source de tension qui active au moins une bobine pour générer un champ magnétique constant.
